# EUROPEAN PATENT SPECIFICATION

(11) **EP 0 360 612 B1**
(45) Date of publication and mention of the grant of the patent: **03.07.1996**
(21) Application number: 89309658.6
(22) Date of filing: 22.09.1989
(51) Int. Cl.: C02F 1/44, B01D 61/00, A23C 9/15, A61L 2/02

(54) **Water purifying system**
Wasserreinigungssystem
Système de purification d'eau

(30) Priority: 23.09.1988 GB 8822470
(43) Date of publication of application: 28.03.1990
(73) Proprietor: HAMPSHIRE ADVISORY AND TECHNICAL SERVICES LIMITED, Upper Shirley Southampton S01 5JD (GB)
(72) Inventor: Wilson, Michael, Dr., London N1 5SU (GB); Monro, Philip, Dr., Southampton SO1 5JD (GB)
(74) Representative: Brooks, Nigel Samuel

(56) References cited:
- EP-A- 0 011 798
- CH-A- 623 233
- US-A- 3 702 820

## Description

The present invention relates to the provision of water free of micro-organisms and in particularly not not exclusively free of the enterotoxins of Vibrio cholerae and Shigella species.

EP-0 011 798 - A1 proposes producing a potable liquid by retaining a water soluble solid in a container provided with a semi-permeable membrane in contact with the solid and contacting the opposite side of the membrane with salt water to allow it to enter the container by osmosis but to prevent the passage of sodium chloride into the container.

According to the invention there is provided a process for producing a non-toxic solution of at least one water soluble solid substantially free of micro-organisms which process comprises retaining the solid or a concentrated solution thereof in a container provided with a selectively permeable membrane in contact with the solid or solution and contacting the opposed side of the membrane with water for a predetermined minimum period of time so as to allow the water to enter the container by osmosis but to prevent the passage into the container of micro-organisms from the water characterised in that the at least one water soluble solid has a molecular weight below the molecular weight cut-off of the membrane.

Preferably, the membrane has a molecular weight cut-off such that micro-organisms and the enterotoxins of V. cholerae and Shigella species are excluded from entering the container.

The invention will be of particular use during periods of emergency due to natural disasters and in other situations where a microbiologically safe supply of water is not readily accessible.

One of the principal causes of sickness and death among small children in the developing countries is diarrhoea. This is due mainly to the inadequacy of the water supply in these countries, eg, in 1985 it was estimated that 59% of the rural population of these countries did not have access to a safe supply of water (Lancet (1987) October 17th p. 890). During periods of emergency caused by natural disasters, epidemics, war etc. access to a safe supply of water becomes even more limited since the usual methods of water purification (filtration and chemical treatment) may not be available and boiling may be impracticable.

The main water-borne microbial pathogens are: Salmonella typhi, Salmonella paratyphi A, B and C, Entamoeba histolytica, Giardia lamblia, Vibrio cholerae, Shigella species, Campylobacter jejuni, Escherichia coli, Vibrio parahaemolyticus, Hepatitis. A virus, rotaviruses and Cryptosporidium and Schistosoma species. In addition, during periods of natural diaster, other infectious diseases can be transmitted via contaminated water. One of the main aims of water treatment processes is to remove such harmful organisms. In addition some of these organisms can form enterotoxins which are liberated from the organism and may, by themselves, cause disease in the absence of the microbe. Such organisms include two of the most important water-borne pathogens, Vibrio cholerae and Shigella species, the causative agents of cholera and bacillary dysentery. The molecular weights of these enterotoxins are 90 kiloDaltons and 82 kiloDaltons respectively. An ideal water-purifying system would therefore also have to ensure that these enterotoxins were removed as well as the microbes themselves.

Prevention, by providing a safe supply of pure water, is the best method of controlling these diseases. Individuals contracting one of these water-borne diseases suffer from a gastro-enteritis which is usually accompanied by diarrhoea. In the very young, the very old, and those debilitated by malnutrition, the diarrhoea results in death due to severe dehydration and electrolyte depletion. However, this can be rectified by "oral rehydration therapy" using an aqueous solution of a mixture of sugars and inorganic salts (Rehydration therapy in diarrhoea, D. Mohalanobis In "Acute enteric infections in children: new prospects for treatment and prevention" Eds, Holme, T., Holmgren, J., Merson, M., Mollby, R, Elsevier, 1981, pp. 303-318). A typical formulation consists of: 23.28 g sucrose, 16.36g glucose, 0.28 g fructose, 1.76 g citric acid, 1.76 g sodium chloride, 1.52g potassium chloride, 1.68 g sodium bicarbonate per litre of solution. However, far simpler formulations are also effective and may contain only glucose or sucrose, sodium chloride, potassium chloride and sodium bicarbonate. The simplest rehydration formulations may contain only glucose and sodium chloride though preferably they also contain potassium chloride. This simple but effective treatment has resulted in a considerable reduction in infant mortality due to gastro-intestinal infections. It should be noted that, apart from typhoid, cholera and some forms of dysentery, antibiotics are not needed for the treatment of most gastro-intestinal infections and, in any case are not generally available in developing countries.

The most important components of an oral rehydration mixture are a sugar, sodium ions and potassium ions. There is some debate as to the optimal concentrations of each of these components and formulations currently being produced have concentrations within the following ranges:
a) for glucose-based mixtures (Bank, S. and Farthing, M.J.G. (1988). Advances in oral rehydration.
Drugs,Supplement 36:p.56 & 83):

| | |
|---|---|
| glucose: | 67-200 mmoles/l |
| sodium ions: | 35-90 mmoles/l |
| potassium ions: | 10-25 mmoles/l |

b) for sucrose-based mixtures (de Zoysa, I. and Lindsay-Smith, E. (1983). In 'Diarrhoea management and oral rehydration therapy in rural homes in Zimbabwe'. Save the Children Fund, London, p.37):

| | |
|---|---|
| sucrose: | 50-149 mmoles/l |
| sodium ions: | 30-100 mmoles/l |

There have recently been suggestions in the literature that the causative agent of viral meningitis, rotaviruses and the oocytes of Cryptosporidium species are not destroyed by chlorination which is a common technique for purifying water from suspect sources.

The present invention provides a simple process for producing clear water free of pathogenic microorganisms and of the enterotoxins of V. cholerae and Shigella species without the need for chemical disinfection or boiling. The process may be used to provide water for drinking or cooking, for medical uses such as in making up and administering drugs by injection or intravenous infusion, for blood or plasma substitutes and for producing oral rehydration compositions. The process may be used in developing countries, particularly during times of natural disasters. It could also be used in other countries by followers of outdoor pursuits such as campers, walkers and mountaineers to supply microbe-free drinking water from streams and rivers and by medical teams denied access to regular supplies of safe water. A particular advantage of the present method is the low weight of the equipment required to produce the desired clean water.

In a particular aspect the method described not only provides a microbe-free and enterotoxin-free supply of water but also provides a solution suitable for oral rehydration therapy.

The present invention therefore provides a process for producing a non-toxic solution of a least one water-soluble solid substantially free of micro-organisms which method comprises retaining the solid or a concentrated solution thereof in a container provided with a selectively permeable membrane in contact with the solid or solution and contacting the opposed side of the membrane with water for a predetermined minimum period of time so as to allow the water to enter the container by osmosis but to prevent the passage into the container of microorganisms from the water.

The selectively permeable membrane permits the passage of water and low molecular weight solutes into and out of the container. When placed in contact with water the membrane therefore allows water to enter the container and dissolve the solid. As the contact time increases more water is drawn into the container by osmotic pressure thereby diluting the solution of the solid. If the solid has a molecular weight below the molecular weight cut-off or exclusion size of the membrane material it will diffuse out of the container and both processes result in a gradual dilution of the solution. Depending upon the choice of solid and the contact time and conditions, the process thus permits the formation of solutions of specified concentration or, by continuing until substantially infinite dilution is achieved, the process permits the production of water almost free of the original water-soluble solid suitable for use, for example, as drinking water. Meanwhile the membrane prevents the ingress of micro-organisms and enterotoxins such as those of V. cholerae and Shigella species so that the water produced will, provided that the water soluble solid and the inside of the container were initially sterile and are not subsequently contaminated during handling, be substantially free of micro-organisms and these enterotoxins. Of course for some applications, such as in producing drinking water for healthy individuals, absolute sterility is not necessary; in such situations the water soluble solid and the inside of the container would be clean and free of harmful amounts of pathogenic micro-organisms. In other applications, such as in producing solutions for intravenous administration, absolute sterility is essential; in these instances the water soluble solid and the interior of the container will be produced in a sterile manner or subsequently sterilised and thereafter maintained sterile until used.

The term "substantially free of micro-organisms" means that the product contains less than 1 viable cell or spore per ml.

The process of the invention may be practised using any appropriate readily available or specially produced selectively permeable membrane material and any water-soluble solid (which may be sterilised if necessary). Typical membrane materials have a molecular weight cut-off in the range of 12-14 kiloDaltons (kD) which will exclude multicellular parasites, protozoa, bacteria, fungi and viruses which may be present in the water outside the tubing and which will exclude enterotoxins such as those of V. cholerae and Shigella species. Some enterotoxins, such as the heat stable enterotoxin of Escherichia coli, have lower molecular weight but could be excluded using a membrane with a molecular weight cut-off of about 4 kD. Such membranes will also exclude endotoxins since these have a molecular weight exceeding 1,000 Kilodaltons. Endotoxins (pyrogens of bacterial origin) are constituents of the walls of Gram-negative bacteria and it is essential that all intravenously-administered solutions are endotoxin-free. Provided that the inside of the semi-permeable membrane and its contents are sterile and endotoxin-free, the invention can therefore be used to provide solutions suitable for intravenous administration.

A variety of semi-permeable materials with different molecular weight cut-offs is available so that there is a wide choice. These materials include:
a, cellulose - widely used to demonstrate the phenomenon of osmosis and in the purification of substances by dialysis.
b, cellophane
c, benzoylated cellulose
d, viscose cellulose
e, collagen.

The preferred material is cellulose since this can be obtained in a very pure form, is non-toxic, is not easily torn or broken when wet and can be left in water for long periods of time without deterioration.

The preferred molecular weight cut-off is 12 - 14 kiloDaltons since this will exclude all microbes and the important enterotoxins (ie, those of Vibrio cholerae, Shigella species, Campylobacter jejuni and the heat-labile enterotoxin of Escherichia coli). In addition it is the most widely used material for dialysis and so is cheap and readily available.

In some applications of the process the solid is present only to secure ingress of water and will be substantially entirely removed before the water is used. In such a situation the nature of the solid is irrelevant, however it will generally be preferred that it has a low molecular weight and that it is non-toxic at least at the final (minimal) concentration. Clearly, if it is to be substantially completely removed by diffusion, the solid must have a molecular weight below the molecular weight cut-off of the membrane.

In other applications the solid is required to be present in the final solution produced by the process. The solid may be retained entirely within the container if its molecular weight is higher than the molecular weight cut-off of the membrane, such as in the case of dextrins, dextrans or certain water soluble glucose polymers [see The Economist, 27th May: 118-120 (1989)]. For those solids which are required to be present in the final solution but have a molecular weight below the cut-off of the membrane, a balance must be struck between the losses to the water outside the container and the time required to secure ingress of sufficient water to establish the desired final concentration.

For production of water with the solid at minimal concentration it is preferable to contact the membrane with running water. Where a particular final concentration of solid is required, this may be achieved using running water but is preferably achieved using a predetermined starting volume of water; in the latter case the water may be static or agitated.

It is a simple matter of trial and error to find the minimum time required to achieve the desired final concentration of solute. It is unlikely that this will be less than 1 minute and convenience dictates that periods longer than 2 days will probably not be of interest. Usually the desired final concentrations can be achieved within a period of from 1 to 24 hours, preferably 2 to 12, for instance 3, 4, 6, 8 or 10 hours.

When it is desired to use more than one solid inside the container, for instance to make up foodstuffs which are solid suspensions, it will be necessary to include at least one soluble solid in order to generate the osmotic pressure which secures ingress of the desired quantity of water.

If two or more solids are included in the container which are water soluble and it is desired to produce a solution of defined concentration of each, the starting amounts of each solid will be adjusted having regard both to the osmotic effect and the rate of diffusion from the container of each. Further details can be seen in the Examples below. In such a case it will be preferable to contact the membrane with a predetermined starting volume of water, when simple trial and error will lead to a determination of the necessary minimum (and maximum) contact time.

A simple way to ensure the final concentration of a solute which has a molecular weight below the molecular weight cut-off of the membrane is to contact the membrane with a predetermined starting volume of water and continue the contact, with or without agitation, until equilibrium is achieved between the concentrations inside and outside the container. Hence sufficient solid is included within the container to achieve the desired concentration in the predetermined starting volume and the predetermined minimum contact time is the time required to establish equilibrium. A similar procedure may be adopted where the water soluble solid is a mixture of solutes one or more of which has a molecular weight below the molecular weight cut-off; contact with the water is maintained until all such solutes have reached equilibrium.

In selecting solid for use in the present invention, it is necessary to take account of the relative water-gathering effect of the various solutes. Since osmotic pressure is governed in part by the number of particles in solution, weight-for-weight a low molecular weight solute will generate a higher osmotic pressure than a high molecular weight solute. However the inward diffusion of water takes a finite time such that the propensity of a low molecular weight solute to diffuse rapidly out of the container will diminish its water gathering effect. For instance sodium and chloride ions diffuse rapidly through cellulose membranes and have little effect on the volume of water gathered by a mixture of sodium chloride and glucose, sucrose or fructose. The sugars actually gather far greater quantities of water as can be seen from the Examples below.

Other water soluble solids which may be included within the container are dried blood products, blood- or plasma-substitutes, components of more complex oral rehydration formulations such as other electrolytes or amino acids and nutritional components such as dried nutritional substances, milk solids and partly dehydrated food containing water-soluble solids. Such solids may be added to the nutritional component if none are present in the component itself. Other favoured solids for water gathering and nutritional purposes are rice powder solutes and rice powder. Nutritional suspensions produced according to the process of the invention may be heated drawing or after the connection of water by the water-soluble solid.

Oral rehydration compositions based on a sugar such as glucose, sucrose or fructose and at least one electrolyte, such as sodium chloride, may be produced by the process of the present invention. Preferably they are produced by contacting the membrane with a predetermined starting volume of water for sufficient time for the electrolyte(s) to equilibrate inside and outside the container and for enough of the sugar to diffuse out to leave the desired sugar and electrolyte concentrations inside the container.

Further solutes, provided that they have a molecular weight below the cut-off of the membrane material, may be introduced into the container by diffusion from the external (dirty) water either during initial collection of water by the water-soluble solid or as a subsequent separate step.

In a particular aspect the invention provides a method which comprises adding a mixture of chemicals of known composition to tubing formed from a semi-permeable material (ie. a material which will permit the passage of molecules below a certain molecular weight- the "molecular weight cut-off"), sealing the tubing at both ends (by tying knots or by other means and then immersing the tubing in water. Water outside the tubing passes through the semi-permeable membrane resulting in a concentrated solution of the mixture inside the tubing. Since this concentrated solution has a higher osmotic pressure than the water outside the tubing, more water diffuses into the tubing so that the total volume of solution inside the tubing gradually increases. At the same time, some of the mixture will gradually pass out of the tubing since the membrane is permeable to the constituents of the mixture. If the semi-permeable material has a molecular weight cut-off of 12 - 14 kiloDaltons then protozoa, bacteria, fungi and viruses which may be present in the water outside the tubing will not be able to pass through into the solution inside. Furthermore, most of the enterotoxins produced by water-borne pathogens are also too large to pass through into the solution. Consequently, there is produced inside the tubing a solution free of all microbes and free of the most important enterotoxins (provided that the inside and the water soluble solid were initially free of such microbes and enterotoxins).

As well as providing a microbe-free supply of water, the invention will also provide a solution suitable for oral rehydration therapy if some attention is given to the duration of immersion of the tubing in the water. The duration of immersion required will depend upon the material of construction of the tubing and the amount and composition of the original mixture contained inside the tubing (see example below).

The container and membrane used in the present invention may take any convenient form. For production of drinking water on a large scale the container may be provided with a membrane of large surface area in a form adapted to contact flowing or static water such as a river or reservoir and the container may be provided with a means for adding further water-soluble solid and/or drawing off purified water. On a smaller scale, such as for use in the production of individual doses of oral rehydration formulation, the container may simply be the membrane in tube form having at least one closed end. Such a container (or at least the closed end thereof) would be immersed in a container such as a bucket or jug of water in order to provide quantities of from a few ml to 1 litre e.g. 50 ml to 500 ml, preferably 100 or 200 ml of oral rehydration formulation after the predetermined minimum immersion period. In other embodiments the container may be provided with an integral outer container for the impure water to be purified and/or with means for drawing off purified water, such as a mouthpiece, drinking tube, septum, hypodermic needle optionally with associated syringe or intravenous infusion set. In further embodiments the container may be reusable and provided with a plurality of water-tight sealed compartments each containing a quantity of water soluble solid. One such compartment would be opened to release the enclosed solid each time the reusable container is used; the compartments may be provided with any conventional means for opening such as screw or click-fit caps, break lines or other weak points or they may simply be opened by cutting or tearing depending upon the material, construction method and final intended use. Such compartments containing quantities of drugs, additional solutes, insoluble materials or liquids may be provided in the containers such that the contents can be released into the purified water once the membrane has been removed from the impure starting water; by this means accurate dosing may be achieved and the loss by diffusion into the starting water of expensive or rare materials may be prevented. For some uses containers including a membrane and at least one water-soluble solid may be provided in continuous web form with tear-off perforations or other means for dividing individual containers from a multi-container web. The containers may also be provided with means for protecting the membrane and/or other fragile, delicate or sterile components during transport and storage. The container may also be provided with suspension means. Means may also be provided for preventing access to the clean solution until the impure water has been removed.

In designing containers provided with (or consisting of) membranes for use in the present invention it is necessary to allow sufficient internal volume to contain the desired final volume of purified water and it is preferable for this to be achieved without generating any back pressure. Such back-pressure will detract from the osmotic pressure exerted by the solutes and, in severe cases, could rupture the membrane leading to loss or contamination of the purified water. Means for obtaining the purified water from the container will be designed with a view to preventing contamination from the environment and particularly from impure water which has been in contact with the membrane (and container).

The container may also be provided with means for indicating that the predetermined minimum contact period has elapsed and/or means for indicating that the interior has ceased to be sterile. This could be achieved for instance for indication of contamination, by applying blue dextran of high molecular weight to the exterior of the membrane whereby contaminated water is coloured. Thus any contamination of the "sterile" solute (if, for example, the membrane has been breached) would be indicated by a blue colouration of the solution. For indication of salt and sugar concentration an indicator strip similar to that used in urine sampling may be used. Such strips may have several sections containing different indicators such that colour indications of (e.g.) pH, glucose, sodium, potassium and chloride concentrations are given. Indicator strips of this kind are available from BDH Limited (Poole, UK).

In one preferred aspect of the invention the preferred shape of the material is as a long narrow tube since this will increase the surface area/volume ratio enabling a more rapid uptake of water. A variety of oral rehydration formulations have been suggested, one of the simplest being the "glucose electrolyte" solution recommended by the World Health Organisation. This consists of: sodium 90 mmol/l, potassium 20 mmol/l, chloride 80 mmol/l, bicarbonate 30 mmol/l, and glucose 111 mmol/l. A more complex formulation consists of: sodium 50 mol/l, potassium 20 mmol/l, chloride 50 mmol/l, bicarbonate 20 mmol/l, glucose 91 mmol/l, fructose 2 mmol/l, sucrose 94 mmol/l and citrate 9 mmol/l.

Flavouring agents may also be added to these formulations as well as high molecular weight soluble proteins and/or polysaccharides and any other components required in the final solution.

Preferably the water purifying system will consist of a length of semi-permeable tubing containing the oral rehydration mixture or other mixture of chemical compounds. The tubing will have been sealed at one end by means of a clip or a knot or by heat-sealing or gluing. The other end will preferably be sealed by means of a clip so that, after the tubing has been immersed in water overnight (or other suitable period of time), it can be re-opened to provide access to the purified water inside. Following immersion of the tubing in the water overnight (or other
suitable period of time) the user will remove the tubing from the water, wipe off any water remaining on the outside of the tubing, remove the clip, and then either pour the contents into a drinking container or drink directly from the tubing using the open end of the tubing as a mouthpiece. The tubing will then preferably be discarded and, since it is biodegradeable, it could be composted or used to improve soil texture. Alternatively, the water purifying system may be supplied in the form of a kit consisting of (1) a length of semi-permeable tubing, sealed at one end and funnelled at the other end (2) a clip for sealing the open end temporarily while the tubing is immersed in water and (3) an oral rehydration mixture or other mixture of chemical compounds. The user will pour the mixture into the tube via the funnelled end, seal the funnelled end with the clip and immerse the tubing in water. After an appropriate period of immersion the user will remove the tubing from the water, wipe off any water adhering to the outside of the tubing, remove the clip and either pour the solution into another drinking container or drink the solution directly from the tubing using the funnelled end as a mouthpiece. The tubing will then preferably be discarded since re-filling with fresh mixture would be technically difficult when the tubing was wet and contamination of the inside of the tubing may take place if care was not exercised.

When the invention is to be used in developed countries to provide only a microbe-free source of water rather than to serve also as a form of rehydration therapy, there exists much greater scope as regards the composition of the mixture to be enclosed within the tubing. For this purpose, for example, the total sugar content can be increased markedly (since the final concentration is not so critical as in the case of oral rehydration therapy) so that the rate of diffusion will correspondingly increase so that the duration of immersion of the tubing can be decreased. In addition, high molecular weight water-soluble proteins and/or polysaccharides may be included in the mixture since these will also increase the rate of uptake of water. Also, flavouring agents to increase the palatability of the drink will be of greater importance.

The invention further provides containers for use in the process described above and the use thereof in producing purified water and medicaments and methods for treating the human or animal body using solutions produced by the process of the invention. Such solutions, containers and methods form a further aspect of the invention.

Particular fileds in which embodiments of the invention may find use include:
A. A method of providing a solution of non-toxic water-soluble solids effective to provide an osmotic pressure free of all micro-organisms and free of the enterotoxins of Vibrio cholerae and Shigella species which method comprises immersing in water a mixture of said compounds enclosed in a sealed drinking container provided with a membrane which is selectively permeable to allow the water to enter the container by osmosis but to prevent the passage into the container of micro-organisms or of molecules of molecular weight greater than 70 kiloDaltons until sufficient water has entered the container to dissolve the salts and diluted them to a concentration which renders the resultant solution drinkable.
B. Such a method wherein the container comprises tubing sealed at each end, one end of which can be re-opened for use as a mouthpiece or to co-operate with a separate mouthpiece for drinking.
C. Such a method wherein the membrane prevents the passage of molecules of molecular weight greater than 12 kiloDaltons.
D. Such a method wherein the membrane is of cellulose or a derivative or regenerated form thereof.
E. Such a method wherein the non-toxic water-soluble salts comprise a mixture with a composition such that it will result in a solution suitable for oral rehydration therapy.
F. A kit for use in a process described herein comprising (1) a mixture of solid chemical compounds for rehydration therapy, (2) a drinking container provided with a membrane as described herein and (3) means for temporarily sealing said container while it is immersed in water.
G. Such a kit wherein the drinking container comprises tubing permanently sealed at one end and wherein clip means are provided for temporarily sealing the other end.
H. Such a kit wherein the mixture of compounds is enclosed within the drinking container.
I. Such a kit which further comprises funnel means co-operable with the container to provide both a mouthpiece for safe drinking from the container and a means for filling the container with the salts.
J. Such a kit wherein the membrane prevents passage of molecules of molecular weight greater than 12 kiloDaltons.
K. Such a kit wherein the membrane is of cellulose or a derivative or regenerated form thereof.
L. The use, in combination, of osmosis to provide water for dissolution of a mixture of water-soluble chemical compounds to form a solution for drinking or for use in rehydration therapy, and of simultaneous dialysis to exclude from the solution micro-organisms or harmful high molecular weight substances, a single membrane being used for the osmosis and dialysis.

The invention will now be illustrated with reference to the figures of the accompanying drawings in which :-
Fig. 1. shows a partially cut-away front view of a container for use in accordance with the invention,
Fig. 2. shows a side view of the device of Fig. 1 and
Fig. 3. shows a front view of an alternative container for use in accordance with the invention.
Fig. 4. is a graph showing the results of Example 2.

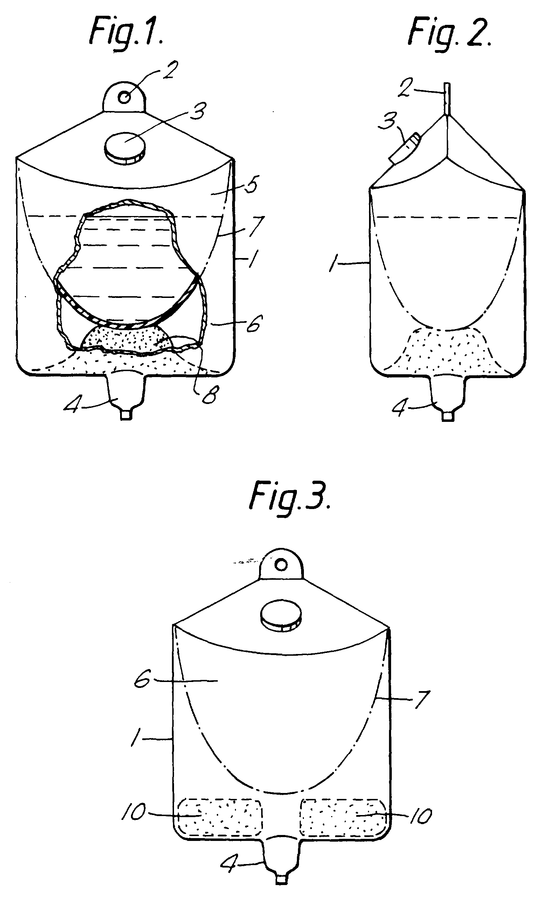

Referring to Fig. 1 the container 1 comprises hanging means 2, a filling spout with closure 3 and a septum 4 for drawing off purified water. Internally the container has two compartments respectively for dirty water 5 and purified water 6 separated by a divider 7 which is a selectively permeable membrane forming a water-tight seal with the walls of the container. Compartment 6 is provided with a quantity of water-soluble solid 8 in contact with the divider 7.

In use dirty water is poured into compartment 5 via the filling spout to a predetermined level and the container is suspended from a suitable hook for a period of time during which the compartment 6 fills with purified water. The residual dirty water may be discarded via the filling spout 3 before purified water is drawn off via the septum 4.

Alternative filling means and means for recovering purified water such as taps or spouts may be provided. It is not essential for the divider between the two compartments to be formed entirely of selectively permeable membrane material provided that a portion of the divider 7 in contact with the solid 8 is such material. The arrangement of the divider 7 within the container is not critical and alternatives such as a horizontal or a diagonal divider are contemplated.

An alternative container for use in accordance with the present invention comprises a container as described in Figs 1 and 2 but, in place of a single quantity of solid 8, as shown in Fig. 3 the container is provided with a plurality of sachets 10 of solid or concentrated solution of solid. The container of Fig 3 is used by bursting one of the sachets 10 and manipulating the solid so as to contact the membrane in the divider 7, then filling the compartment 6 with dirty water and recovering purified water as previously described. Once the residual dirty water has been discarded and the purified water recovered, the container may be reused by bursting a second sachet 10 and proceeding with a new volume of dirty water. Additional sachets may be provided each containing solid or liquid components, such as drugs, for bursting and release of the solid or liquid components into the purified water. The compartment for purified water may be sub-divided into a plurality of sealed compartments each provided with a portion or portions of water-soluble solid, a semi-permeable membrane and means for recovering water.

Different sachet may be coded, for instance colour coded, with each containing a different substance to be added.

Many further variants of the container are contemplated for use in accordance the invention.

The invention will further be illustrated by the following Examples.

### EXAMPLES

### Example 1

The following experiments have been conducted to establish the conditions for producing an oral rehydration formulation by the process of the invention. Unless otherwise stated, the glucose determinations were performed using a Technicon (Trade Mark) RA 1000 (Technicon, Basingstoke, UK) while the sodium and potassium ion concentrations were carried out using an IL 343 Flame Photometer (Instrumentation Laboratories Ltd, Warrington, UK). In many cases, the solutions to be assayed first had to be diluted to a considerable extent.

### Experiment 1

Portions of 40g of the following formulation: 20 parts glucose, 5.25 parts sodium chloride, 1.5 parts potassium chloride (all parts by weight) were placed in sections of 38mm (1 1/2") or 29mm (1 1/8") diameter cellulose dialysis tubing which was then sealed at both ends. The tubing sections were each immersed in flowing water at 17°C or 30°C. Sections of 29mm tubing were immersed in 1 litre of static water at 17°C or 30°C.

The results are shown in Tables 1 to 6.

**Table 1.**

| 17°C flowing, 38mm diameter tubing. | | | | |
|---|---|---|---|---|
| Time (h) | Volume accumulated (ml) | glucose mmoles/l | [Na⁺] mmoles/l | [K⁺] mmoles/l |
| 3.5 | 108 | 960 | 290 | 25.5 |
| 4.5 | 126 | 676 | 121 | 8.4 |
| 5.5 | 138 | 488 | 64 | 3.6 |
| 6.5 | 144 | 338 | 38 | 1.8 |
| 7.5 | 148 | 328 | 25 | 1.1 |
| 8.25 | 151 | 224 | 16 | 0.6 |

**Table 2.**

| 17°C flowing, 29mm diameter tubing | | | | |
|---|---|---|---|---|
| Time (h) | Volume accumulated (ml) | glucose mmoles/l | [Na⁺] mmoles/l | [K+] mmoles/l |
| 2.5 | 110 | 902 | 286 | 37.8 |
| 3.5 | 132 | 612 | 121 | 12.4 |
| 4.5 | 152 | 376 | 43 | 3 |
| 5.5 | 166 | 366 | 18 | 0.9 |
| 6.5 | 173 | 166 | 9 | 0.4 |
| 7.5 | 178 | 128 | 5 | 0.2 |
| 8.25 | 180 | 78 | 3 | 0.1 |

**Table 3.**

| 30°C flowing, 29mm diameter tubing | | | | |
|---|---|---|---|---|
| Time (h) | Volume accumulated (ml) | glucose mmoles/l | [Na⁺] mmoles/l | [K⁺] mmoles/l |
| 2.5 | 138 | 478 | 67 | 6.4 |
| 3.5 | 160 | 272 | 19 | 1.2 |
| 4.5 | 176 | 124 | 6 | 0.3 |
| 5.5 | 178 | 80 | 3 | 0.3 |
| 6.5 | 177 | 146 | 3 | 0.2 |
| 7.5 | 178 | 96 | 3 | 0.3 |
| 8.25 | 178 | 170 | 2 | 0.2 |

**Table 4.**

| 30°C flowing, 38mm diameter tubing | | | | |
|---|---|---|---|---|
| Time (h) | Volume accumulated (ml) | glucose mmoles/l | [Na⁺] mmoles/l | [K⁺] mmoles/l |
| 2.5 | 130 | 128 | 127 | 13.6 |
| 3.5 | 150 | 128 | 45 | 3.4 |
| 4.5 | 162 | 194 | 11 | 0.5 |
| 5.5 | 168 | 86 | 4 | 0.2 |
| 6.5 | 174 | 78 | 3 | 0.2 |
| 7.5 | 174 | 102 | 3 | 0.2 |
| 8.25 | 174 | 100 | 2 | 0.1 |

**Table 5.**

| 17°C, fixed external volume of water | | | | |
|---|---|---|---|---|
| Time (h) | Volume accumulated (ml) | glucose mmoles/l | [Na⁺] mmoles/l | [K⁺] mmoles/l |
| 3.5 | 112 | 954 | 376 | 68 |
| 4.5 | 124 | 704 | | 44.5 |
| 5.5 | 136 | 562 | 194 | 36.2 |
| 6.5 | 144 | 456 | 170 | 33.5 |
| 7.5 | 150 | 382 | 155 | 32 |
| 8.5 | 151 | 320 | 116 | 25.5 |
| 24 | 166 | 120 | 97 | 22 |

**Table 6.**

| 30°C fixed external volume of water. | | | | |
|---|---|---|---|---|
| Time (h) | Volume accumulated (ml) | glucose mmoles/l | [Na] mmoles/l | [K] mmoles/l |
| 2.5 | 116 | 462 | 288 | 51.2 |
| 3.5 | 132 | 576 | 190 | 35.7 |
| 4.5 | 142 | 428 | 166 | 33 |
| 5.5 | 148 | 366 | 149 | 31 |
| 6.5 | 152 | 296 | 145 | 31.5 |
| 7.5 | 154 | 280 | 149 | 33 |
| 8.5 | 152 | 224 | 120 | 27.9 |
| 24 | 154 | 116 | 125 | 28.7 |

### Comments

Use of this particular formulation in running water does not provide the necessary balance of glucose/sodium ions/potassium ions for oral rehydration therapy. By immersing for 8 h or more it provides a sterile glucose solution for drinking purposes (Tables 1 - 4).

Use of this formulation in a fixed external volume of water provides a solution with approximately the correct glucose/salts balance for oral rehydration (Tables 5 and 6).

Increasing the temperature from 17°C to 30°C appears to increase the rate of uptake of water and the rate which the glucose/salts equilibrium is reached (Tables 5 and 6).

The diameter of the tubing appears to affect the rate of uptake of water and the composition of the resulting solution. The smaller diameter tubing has a greater rate of water uptake and glucose/salt loss than the larger diameter (Tables 1 and 2). This is presumably due to the greater surface area/volume ratio.

### Experiment 2

The purpose of this was to study the effect of varying the external volume of water on both the uptake of water and the composition of the resulting solution.

Portions of 10g of the same glucose/sodium chloride/potassium chloride mixture as used in Experiment 1 were added to 22 cm lengths of 29mm diameter tubing and the tubing immersed in 50 to 500 ml of water at 30°C. The internal volume and concentrations were measured at 24 hours. The results are shown in Table 7.

**Table 7.**

| initial external volume (ml) | Volume taken up (ml) | glucose mmoles/l | Na⁺ mmoles/l | K⁺ mmoles/l |
|---|---|---|---|---|
| 500 | 45 | 118 | 60 | 14 |
| 450 | 45 | 103 | 80 | 18 |
| 400 | 45 | 123 | 77 | 16 |
| 350 | 47 | 110 | 101 | 22 |
| 300 | 46 | 115 | 117 | 26 |
| 250 | 45 | 170 | 137 | 31 |
| 200 | 44 | 205 | 171 | 39 |
| 150 | 42 | 301 | 228 | 52 |
| 100 | 38 | 422 | 321 | 73 |
| 50 | 30 | 785 | 820 | 179 |
| 40 | 27 | 968 | 920 | 199 |

### Comments

Varying the volume of water in which the formulation is immersed affects both the total volume of water taken up and the composition of the resulting solution. Hence, it is possible to obtain a solution of known composition by immersing the formulation in an appropriate volume of water. From the above results it can be seen that for oral rehydration therapy, a solution of suitable composition can be obtained by immersing 10 g of the above formulation in from 400 to 500 ml of water.

### Experiment 3

The following experiment was performed to study the effect of varying the composition of the mixture on the resulting composition of the solution.

A new formulation was used containing 5.22 parts sodium chloride, 1.125 parts potassium chloride and various amounts of glucose ranging from 16 parts to 32 parts by weight. Portions of this mixture were added to 29mm tubing and incubated in 1 litre water at 30°C. The internal volume and concentrations were measured at 24 hours and the results are shown in Table 8.

**Table 8.**

| Glucose (g) | Volume (ml) | glucose mmoles/l | Na⁺ mmoles/l | K⁺ mmoles/l |
|---|---|---|---|---|
| 16 | 102 | 65 | 88 | 15 |
| 18 | 110 | 79 | 81 | 14 |
| 20 | 117 | 136 | 87 | 15 |
| 22 | 124 | 143 | 85 | 14 |
| 24 | 133 | 147 | 80 | 13 |
| 26 | 142 | 169 | 86 | 14 |
| 28 | 152 | 192 | 92 | 15 |
| 30 | 160 | 192 | 82 | 14 |
| 32 | 170 | 222 | 81 | 14 |

The experiment was repeated using a mixture of 36 g glucose, 10.44 g sodium chloride and 2.25 g potassium chloride in 29mm tubing immersed in 2 litres water at 30°C for 18 and 24 hours. The results are shown in Table 9.

**Table 9.**

| Time (h) | Volume (ml) | glucose mmoles/l | Na⁺ mmoles/l | K⁺ mmoles/l |
|---|---|---|---|---|
| 18 | 210 | 123 | 88 | 16 |
| 24 | 214 | 106 | 90 | 15 |

### Comments

When a mixture containing between 16 g and 20 g of glucose, 5.22 g sodium chloride and 1.125g potassium chloride is immersed in 1 litre of water, solutions suitable for oral rehydration therapy are produced (Table 8).

Larger volumes of solutions suitable for oral rehydration therapy can be obtained using larger amounts of the mixture providing that the volume of water in which it is immersed is increased (Table 9).

### Experiment 4

Experiment 3 was repeated using a mixture of 18 g glucose, 5.22 g sodium chloride and 1.125 g potassium chloride in 29mm tubing incubated in 1 litre of water at 4°C. Glucose concentration was determined using a diagnostic kit purchased from Sigma Ltd (Poole, UK). Sodium ion concentrations were determined using a sodium-sensitive electrode (Whatman International Ltd, Maidstone, UK). The results are shown in Table 10.

**Table 10.**

| Time (h) | Volume (ml) | Glucose mmoles/l | Na⁺ mmoles/l |
|---|---|---|---|
| 24 | 118 | 115 | 65 |

### Comments

This demonstrates that solutions suitable for oral rehydration therapy can be obtained within 24 h even when the temperature of the water is as low as 4°C.

### Experiment 5

Although solutions containing glucose, sodium chloride and potassium chloride are recommended for oral rehydration therapy, simpler formulations containing only sucrose and sodium chloride have also been shown to be effective.

Portions of 50 g sucrose and 120 g sodium chloride were placed inside sections of 38mm or 83mm (3 1/4") tubing and immersed in flowing water at room temperature for various times and the internal volume and concentrations were measured. Sucrose concentrations were determined using the phenol/sulphuric acid method of Dubois et al., Analytical Chemistry, 39 ; 350-356 (1956). Sodium ion concentrations were determined using a sodium-sensitive electrode (Whatman International Ltd). The results are shown in Table 11.

**Table 11.**

| Tubing | Time (h) | Volume (ml) | sucrose mmoles/l | Na⁺ mmoles/l |
|---|---|---|---|---|
| 38mm | 16 | 670 | 80 | 17 |
| | 20 | 695 | | 9 |
| | 24 | 715 | 19 | 7 |
| | 40 | 755 | 13 | 9 |
| | | | | |
| 83mm | 16 | 650 | 96 | 46 |
| | 20 | 675 | 57 | 17 |
| | 24 | 680 | 46 | 12 |
| | 40 | 715 | 19 | 12 |

### Comments

The rate of water uptake and composition of the solution was dependent on the diameter of tubing used. Using 83mm tubing large quantities (650 ml) of a solution suitable for oral rehydration therapy was obtained after 16 h (Table 11). By extending the period of immersion beyond 16 h this method could also be useful for obtaining large quantities of sterile sucrose-containing solutions for drinking and other purposes. Water uptake by sucrose is appreciably more effective than with glucose.

### Experiment 6

The purpose of this experiment was to study the effect of immersing sucrose-containing mixtures in fixed volumes of water. Portions of the following mixtures were placed inside sections of 32 mm (1 1/4") tubing and immersed in water at 30°C for various times and the internal volume and concentrations were measured. The results are shown in Table 12.

| | | | |
|---|---|---|---|
| A | 20g sucrose, | 5.22g NaCl | 1.13g KCl |
| B | 20g sucrose | 7.83g NaCl | 1.69g KCl |
| C | 20g sucrose | 10.44g NaCl | 2.25g KCl |
| D | 30g sucrose | 5.22g NaCl | 1.13g KCl |

**Table 12**

| Mixture | Initial volume | Time (h) | Volume (ml) | sucrose mmoles/l | Na⁺ mmoles/l | K⁺ mmoles/l |
|---|---|---|---|---|---|---|
| A | 1 litre | 8 | 138 | 128 | 77 | 13 |
| | | 17 | 162 | | 89 | 15 |
| | | 24 | 162 | 73 | 73 | 12 |
| | | | | | | |
| B | 1.5 litres | 8 | 156 | 126 | 86 | 14 |
| | | 17 | 170 | | 80 | 13 |
| | | 24 | 174 | 52 | 76 | 13 |
| | | | | | | |
| C | 2 litres | 8 | 168 | 110 | 87 | 14 |
| | | 17 | 182 | | 85 | 14 |
| | | 24 | 186 | 37 | 84 | 14 |
| | | | | | | |
| D | 1 litre | 8 | 188 | 186 | 86 | 14 |
| | | 17 | 228 | | 78 | 13 |
| | | 24 | 228 | 107 | 91 | 15 |

### Comments

Solutions with a composition suitable for oral rehydration therapy can be obtained after only 8h immersion in either 1, 1.5 or 2 litres of water (Table 12). Water uptake by sucrose is appreciably more effective than that attained with glucose.

### Experiment 7

The purpose of the experiments described below was to study the ability of the membrane to exclude particles present in the external solution.

38mm tubing containing 40 g glucose/Sodium chloride/potassium chloride mixture was immersed in static water containing Pseudomonas aeruginosa and Blue Dextran (0.2 g/litre, mw 2 x 10⁶D).
Final OD of liquid external to tubing: A₆₂₀ = 0.241
Final OD of liquid inside tubing : A₆₂₀ = 0

Pseudomonas content of liquid outside tubing = 45 x 10⁶ organisms per ml. After 8 1/4 h of immersion no Pseudomonas could be detected inside tubing. The assay method used could detect bacteria at 1 organism per ml.

### Comments

The membrane succesfully excluded blue dextran (molecular weight 2 x 10⁶) and Pseudomonas aeuginosa. Since pathogenic organisms are larger than the blue dextran molecules these results suggest that harmful bacteria and viruses will be excluded by the membrane.

### Experiment 8

The purpose of this experiment was to study how the individual components of oral rehydration therapy mixtures behave when contained in a semipermeable membrane.
100 mmoles portions of glucose (18g), sodium chloride (5.8g) or potassium chloride (7.4g) were placed in sections of 29mm tubing and each section of tubing was immersed in 1 litre of water at 30°C. The internal volume and internal and external concentrations of glucose, sodium chloride and potassium chloride were measured. The results are shown in Table 13.

**Table 13**

| Time (h) | glucose mmoles/l | | | NaCl mmoles/l | | | KCl mmoles/l | | |
|---|---|---|---|---|---|---|---|---|---|
| | vol (ml) | in | out | vol (ml) | in | out | vol (ml) | in | out |
| 8 | 80 | 160 | 100 | 16 | 111 | 120 | 11 | 110 | 102 |
| 24 | 82 | 109 | 103 | 16 | 110 | 110 | 11 | 100 | 102 |

### Comments

These results show that most of the water uptake takes place during the first 8 h of immersion and that glucose has the greatest water-uptake potential per mole of the compounds tested.

Both the sodium chloride and the potassium chloride rapidly equilibrate (within 8h) with the solution external to the membrane whereas the glucose takes appreciably longer to reach equilibrium.

By varying the volume of the external solution and the amount of substance originally inside the membrane, this system could be used to obtain sterile glucose and saline solutions of almost any desired composition. As the majority of water is drawn in by the sugar and there is rapid equilibrium of the salts this has two very important practical implications. Firstly it means that there is little chance (when the invention is used with potassium salts in either oral or injection therapy) of having the salt at a dangerous concentration so long as at least half of the required water has entered the bag. Secondly it means that a sugar, or dextran or even "pure" water can be immersed in an appropriate saline solution and the salts will enter by diffusion. This will allow easy additions on a simple formulation to be produced.

### Experiment 9

The ability of other water-soluble compounds to take up water was investigated as follows:

Portions of (a) 1 g of dextran (clinical grade, mw 60-90,000), (b) 10 g of dextrin (type 3), (c) 10g dextrin and 5 g sucrose, and (d) 5 g sucrose were placed in sections of 29mm tubing and the tubing immersed in flowing water (room temp). The internal volume was measured and the results are shown in Table 14

**Table 14**

| Vol after 18 h | after 24h | |
|---|---|---|
| dextran = 18ml (18 ml/g) | 20ml (20 ml/g) | (0.95 dry wt was recovered from tubing) |
| sucrose = 39ml (8 ml/g) | 39ml (8 ml/g) | |
| dextrin = 66ml (7 ml/g) | 76ml (8 ml/g) | |
| dextrin + sucrose = 86ml (6 ml/g) | 98ml (7 ml/g) | |

### Comments

On a weight basis, dextran appears to be the most effective at taking up water.

### Experiment 10

Dextran solutions containing saline are commonly used as plasma substitutes administered by intravenous infusion. The purpose of this experiment was to determine whether dextran could be used to draw in water through a semipermeable membrane and so obtain microbe-free solutions.

Portions of (a) 2g of dextran (clinical grade, molecular weight approx. 70,000) or (b) 2 g of dextran and 9 g of sodium chloride were placed inside sections of 29mm tubing and the tubing sections were immersed in 1 litre water at 20°C. A further portion of 2 g of dextran in a section of 29mm tubing was immersed in 1 litre of saline (9g per litre). A 40 ml aliquot of a 5% dextran solution (prepared as described in experiment 9 above) was placed in 29mm tubing which was then immersed in saline (9 g per litre).

The volume of the resultant solutions and their sodium ion content were determined (using a Na⁺ ion electrode) after various times of immersion. The results are shown in Table 15.

**Table 15**

| | time (h) | Volume (ml) | Na⁺ (mmoles/l) |
|---|---|---|---|
| Dextran in water | 3 | 10 | |
| | 5.5 | 13 | |
| | 7 | 15 | |
| | 8.5 | 17 | |
| | 10.5 | 18 | |
| | 13.5 | 21 | |
| | 17 | 24 | |
| | 24 | 28 | |
| | 27 | 29 | |
| | 30 | 30 | |
| | 33 | 32 | |
| | 96 | 40 | |
| | | | |
| Dextran + NaCl in water | 2 | 29 | 520 |
| | 3 | 30 | 200 |
| | 5 | 32 | 160 |
| | 7 | 34 | 160 |
| | 10 | 35 | 160 |
| | 12 | 36 | 160 |
| | 24 | 40 | 160 |
| | 28 | 42 | 160 |
| | 31 | 43 | 160 |
| | 48 | 49 | 160 |
| | | | |
| Dextran in saline | 3 | 12 | - |
| | 5 | 14 | 160 |
| | 7 | 17 | 160 |
| | 10 | 20 | 160 |
| | 12 | 21 | 160 |
| | 24 | 28 | 160 |
| | 28 | 30 | 160 |
| | 31 | 31 | 160 |
| | 48 | 37 | 160 |
| | | | |
| Dextran solution in saline | 1 | 40 | 100 |
| | 2 | 40 | 130 |
| | 3 | 40 | 130 |
| | 4 | 40 | 160 |
| | 6 | 41 | 160 |
| | 8 | 41 | 160 |
| | 11 | 43 | 160 |
| | 13 | 43 | 160 |
| | 24 | 46 | 160 |
| | 28 | 47 | 160 |
| | 31 | 48 | 160 |
| | 48 | 53 | 160 |

### Comments

Dextran is effective at drawing in water across a semipermeable membrane although, unlike the other solutes tested, the uptake requires a long time before "saturation" is reached. A further point of difference from sucrose, glucose and sodium chloride is that the dextran cannot itself cross the semipermeable membrane (its molecular weight is 70,000) and therefore is totally retained within the tubing. This was verified by taking a 10 ml sample from the tubing and evaporating this to dryness, the dry weight of the recovered dextran showed that all of it had been retained in the bag. Immersion of a mixture of 2 g of dextran and 9 g of sodium chloride in 1 litre of water for 24 h resulted in a solution with the ideal composition for intravenous infusion, namely 5% dextran and 0.9% sodium chloride. Such a solution could also be achieved by immersing a 5% dextran solution (prepared as described above) in 0.9% sodium chloride for 4 h. The approach could also be used to prepare dextran-based solutions supplemented with other solutes including, or course, a variety of drugs.

### Example 2

A mixture suitable for oral rehydration therapy was prepared containing the following chemicals (per 100 g of mixture)
3.18g sodium chloride
2.75g potassium chloride
3.04g sodium bicabonate
3.18g citric acid
29.55g glucose
58.31g sucrose

11g of this mixture was then poured into a 30 cm length of cellulose dialysis tubing with a diameter of 4.2 cm and a molecular weight cut-off of 12-14 kiloDaltons (Medicell Ltd., Islington, London). The tubing was then sealed by tying a knot in each end (after carefully moistening each of the ends) and immersed in a large container through which was flowing a constant stream of tap water. After various time intervals the tubing was removed from the water and the volume of solution inside the tubing was measured. The solution was then poured back into the tubing which was then re-sealed and re-immersed in the flowing water.

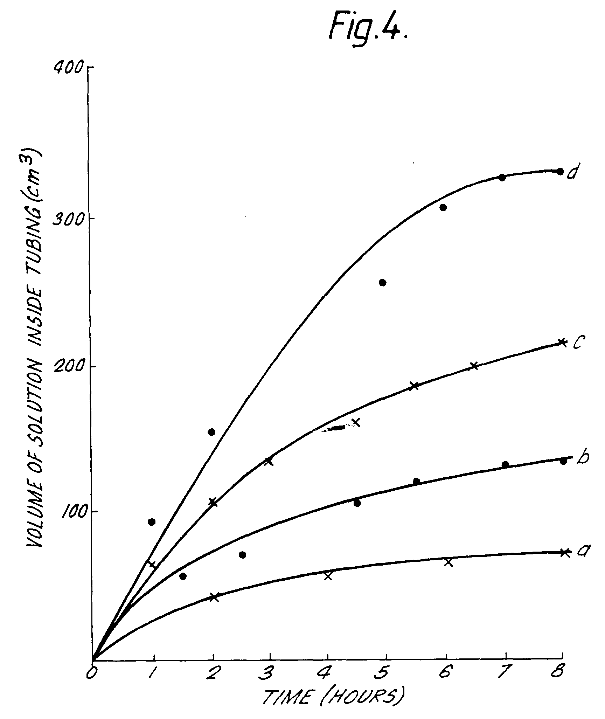

After 8 hours immersion in the flowing water the solution within the tubing was analysed for its total sugar content by means of a pocket refractometer (Bellingham and Stanley Ltd., Tunbridge Wells, UK). A graph was then plotted of volume of water within the tubing against time (Figure 4, curve a).

The experiment was then repeated using increasing quantities of the mixture and, where appropriate, longer lengths of the same type of dialysis tubing (Figure 4). Curve b of Figure 4 describes the uptake of water when the initial quantity of the mixture was 22 g, when the initial quantity of mixture was 33 g curve c was obtained, and when the initial quantity of mixture was 55 g curve d was obtained.

The effect of the quantity of mixture used on the final volume of solution obtained and the final sugar concentration is shown in Table 16. It can be seen from Figure 4 that for each quantity of mixture used the volume of water diffusing into the tubing increases at first then levels off tending towards a maximum value.

An immersion period of 8 hours was chosen because this is the average duration of sleep for an adult. The tubing could therefore be immersed in water before going to sleep and on waking the solution inside the tubing would be at a concentration suitable for oral rehydration therapy.

In the table below it can be seen that the volume of water "purified" (i.e, taken up into the dialysis tubing) increases as the initial quantity of mixture used increases. The final concentration of sugar was, in each case, between 3% and 4.75% after the 8 hour immersion period.

**Table 16**

| Quantity of Mixture in tubing | Volume of Solution inside tubing after 8 h | Concentration of sugar in solution after 8 h |
|---|---|---|
| (g) | (cm³) | (%) |
| 11 | 71 | 3.0 |
| 22 | 136 | 4.5 |
| 33 | 216 | 4.0 |
| 55 | 332 | 4.75 |

## Claims

1. A process for producing a non-toxic solution of at least one water soluble solid substantially free of micro-organisms which process comprises retaining the solid or a concentrated solution thereof in a container provided with a selectively permeable membrane in contact with the solid or solution and contacting the opposed side of the membrane with water for a predetermined minimum period of time so as to allow the water to enter the container by osmosis but to prevent the passage into the container of micro-organisms from the water characterised in that the at least one water soluble solid has a molecular weight below the molecular weight cut-off of the membrane.

2. A process as claimed in claim 1, characterised in that the membrane has a molecular weight cut-off such that micro-organisms and the enterotoxins of V. cholerae and Shigella species are excluded from entering the container.

3. A process as claimed in claim 1 or claim 2, characterised in that at least one other water soluble solid is retained within the container, the at least one other water soluble solid having a molecular weight above the molecular weight cut-off of the membrane.

4. A process as claimed in claim 1, claim 2 or claim 3, characterised in that at least one of the water soluble solids is selected from sugars, water soluble glucose polymers, water soluble polysaccharides, rice powder, electrolyte salts and amino acids.

5. A process as claimed in claim 4, characterised in that at least one of the water soluble solids is a sugar selected from glucose, dextrose, fructose and sucrose.

6. A process as claimed in any preceding claim, characterised in that the membrane is contacted with flowing water.

7. A process as claimed in claim 6, characterised in that the membrane is contacted with flowing water for sufficient time for substantially all the water soluble solids having a molecular weight below the molecular weight cut-off of the membrane to diffuse out of the container.

8. A process as claimed in claim 6, characterised in that all the water soluble solids have a molecular weight below the molecular weight cut-off of the membrane and in that the membrane is contacted with flowing water for sufficient time for substantially all the water soluble solids to diffuse out of the container for producing potable water substantially free of water-soluble solids.

9. A process as claimed in any one of claims 1 to 5, characterised in that the membrane is contacted with a predetermined volume of static or agitated water.

10. A process as claimed in claim 9, characterised in that the membrane is contacted with water for sufficient time for at least one of the water soluble solids having a molecular weight below the molecular weight cut-off to diffuse out of the container until the concentration of the solid inside the container is in equilibrium with the concentration outside the container.

11. A process as claimed in any one of claims 1 to 5 or claim 9 or claim 10, characterised in that the water soluble solids constitute a mixture suitable for producing a formulation for oral rehydration therapy and the membrane is contacted with water for a predetermined time so as to form a formulation for oral rehydration therapy within the container.

12. A process as claimed in any one of claims 1 to 5 or claim 9 or claim 10, characterised in that the process produces a solution of a drug for injection, preferably free from endotoxins and pyrogens.

13. A process as claimed in any one of claims 1 to 5 or claim 9 or claim 10, characterised in that the process is used for rehydrating dried blood products or producing a blood- or plasma-substitute solution for intravenous infusion, preferably free from endotoxins and pyrogens.

14. A process as claimed in any one of claims 1 to 5 claim 9 or claim 10, characterised in that the process is used for producing a nutritional suspension, the process comprising retaining in the container a dried nutritional substance, milk solids or partially dehydrated food substance containing at least one water soluble solid in contact with the membrane.

15. A process for rehydrating dried blood products or producing a blood- or plasma-substitute solution substantially free of micro-organisms for intravenous infusion, the process comprising:
retaining the solids of blood products or blood- or plasma-substitute or a concentrated solution thereof in a container provided with a selectively permeable membrane in contact with the solid or solution, the solids or concentrated solution thereof including at least one water soluble solid which has a molecular weight below the molecular weight cut-off of the membrane; and
contacting the opposed side of the membrane with water for a predetermined minimum period of time so as to allow the water to enter the container by osmosis but to prevent the passage into the container of micro-organisms, and preferably endotoxins and pyrogens, from the water.

16. A process for producing a solution of drug for injection substantially free of micro-organisms, the process comprising:
retaining the drug solids or a concentrated solution thereof in a container provided with a selectively permeable membrane in contact with the solid or solution, the solids or concentrated solution thereof including at least one water soluble solid which has a molecular weight below the molecular weight cut-off of the membrane; and
contacting the opposed side of the membrane with water for a predetermined minimum period of time so as to allow the water to enter the container by osmosis but to prevent the passage into the container of micro-organisms, and preferably endotoxins and pyrogens, from the water.

17. A process for producing a water for medical purposes, in particular for making up and administering drugs by injection or intravenous infusion, the process comprising:
retaining at least one water soluble solid or a concentrated solution thereof in a container provided with a selectively permeable membrane in contact with the solid or solution, the solid(s) or concentrated solution thereof including at least one water soluble solid which has a molecular weight below the molecular weight cut-off of the membrane; and
contacting the opposed side of the membrane with water for a predetermined minimum period of time so as to allow the water to enter the container by osmosis but to prevent the passage into the container micro-organisms, and preferably endotoxins and pyrogens, from the water with which the membrane is contacted.

18. A process for producing a solution for oral rehydration therapy of water soluble solids substantially free of micro-organisms which process comprises retaining the solids or a concentrated solution thereof in a container provided with a selectively permeable membrane in contact with the solids or their concentrated solution and contacting the opposed side of the membrane with water for a predetermined minimum period of time so as to allow the water to enter the container by osmosis but to prevent the passage into the container of micro-organisms from the water characterised in that:
the water soluble solids are adapted to produce an oral rehydration therapy solution;
at least one of the water soluble solids is selected from electrolyte salts, having a molecular weight of below the molecular weight cut-off of the membrane;
a predetermined quantity of the water soluble solids are provided in the container; and
the membrane is contacted with a predetermined volume of static or agitated water.

19. A process as claimed in claim 18, characterised in that another of the water soluble solids is selected from sugars, water soluble glucose polymers, water soluble polysaccharides and rice powder.

20. A process as claimed in any preceding claim, characterised in that the membrane is selected from cellulose, cellophane, benzoylated cellulose, viscose cellulose and collagen.

21. A container for use in producing solutions by the process of claim 1, the container having a first compartment (6) with a selectively permeable membrane and containing the solid (8) or a concentrated solution thereof, characterised in that
the container comprises a second compartment (5), in that
the first and second compartments are separated by a member (7) including the selectively permeable membrane and in that
the at least one water soluble solid has a molecular weight below the molecular weight cut-off of the membrane.

22. A container as claimed in claim 21, characterised in that the membrane is selected from cellulose, cellophane, benzoylated cellulose, viscose cellulose and collagen.

23. A container for use in producing an oral rehydration therapy solution by the process of claim 18, the container having: a first compartment (6) with a selectively permeable membrane and containing the predetermined quantity of the water soluble solids (8) or a concentrated solution thereof, characterised in that the container comprises:
a second compartment (5) for containing a predetermined quantity of water,
means (3) for introducing the water into the second compartment,
a member (7) including the selectively permeable membrane separating the first and second compartments and
means (4) for withdrawing the said solution from the first compartment and in that
the at least one water soluble solid has a molecular weight below the molecular weight cut-off of the membrane.

24. A container as claimed in claim 21, claim 22 or claim 23 characterised in that the first compartment comprises a plurality of burstable compartments (10), each containing a predetermined quantity of the water soluble solids or a concentrated solution thereof, the container being reusable after a first of the compartments has been burst to place its contents into the first compartment, water has permeated into the first compartment to form a solution and been emptied therefrom.

25. A container for producing potable, non-toxic water substantially free of micro-organisms by the process of claim 6, the container having a selectively permeable membrane and containing in contact with the membrane the water soluble solid or solids, or a concentrated solution thereof, characterised in that the at least one or all of the said solid(s) has/have a molecular weight below the molecular weight cut-off of the membrane.

26. A container as claimed in any one of claims 21 to 25, characterised in that the first compartment or the second compartment comprises means for indicating when the solution has reached its intended formulation.

27. A container for producing a nutrional suspension substantially free of micro-organisms by the process of claim 14, characterised in that the container has a dried nutritional substance, milk solids or partially dehydrated food substance containing at least one water soluble solid in contact with the membrane, the or one of soluble solid(s) having a molecular weight below the molecular weight cut-off of the membrane.

28. A container for rehydrating dried blood products or producing a blood- or plasma-substitute solution substantially free of micro-organisms, and preferably endotoxins and pyrogens, for intravenous infusion by the process of claim 15, the container having a selectively permeable membrane and containing the solids of blood products or blood- or plasma-substitute or a concentrated solution thereof and at least one water soluble solid having a molecular weight below the molecular weight cut-off of the membrane in contact with the membrane.

29. A container for producing a solution of drug for injection substantially free of micro-organisms, and preferably endotoxins and pyrogens, by the process of claim 16, the container having a selectively permeable membrane and containing soluble solid(s) of the drug or a concentrated solution thereof in contact with the membrane, at least one of the soluble solid(s) having a molecular weight below the molecular weight cut-off of the membrane.

30. A container for producing a water for medical purposes, in particular making up and administering drugs by injection or intravenous infusion substantially free of micro-organisms, and preferably endotoxins and pyrogens, by the process of claim 17, the container having a selectively permeable membrane and containing the at least one water soluble solid or a concentrated solution thereof in contact with the membrane, the at least one water soluble solid having a molecular weight below the molecular weight cut-off of the membrane.

31. Use of a container as claimed in any one of claims 21 to 26 or claim 29 or claim 30 for the preparation of a potable water or a potable solution or a medicament for use in treatment of the human or animal body or water for making up and administering a drug by injection or infusion, the preparation being by the method of claims 1, 16 or 17 respectively.

## Patentansprüche

1. Verfahren zum Herstellen einer nicht-toxischen Lösung wenigstens eines wasserlöslichen Feststoffs, der frei von Mikroorganismen ist, indem man den Feststoff oder eine konzentrierte Lösung desselben in einen Behälter hält, der mit einer selektiv permeablen Membran versehen ist, die mit dem Feststoff oder der Lösung in Berührung ist, und die entgegensetzte Seite der Membran über eine vorbestimmte minimale Zeitdauer mit Wasser in Berührung bringt, um es dem Wasser zu ermöglichen, durch Osmose in den Behälter einzudringen, aber den Durchgang von Mikroorganismen aus dem Wasser in den Behälter zu verhindern, **dadurch gekennzeichnet**, daß der wenigstens eine wasserlösliche Feststoff ein Molekulargewicht unterhalb der Molekulargewichtsausschlußgrenze der Membran hat.

2. Verfahren nach Anspruch 1, dadurch gekennzeichnet, daß die Membran eine solche Molekulargewichtsausschlußgrenze hat, daß Mikroorganismen und die Enterotoxine der Spezies V. cholerae und Shigella am Eintritt in den Behälter gehindert sind.

3. Verfahren nach Anspruch 1 oder 2, dadurch gekennzeichnet, daß wenigstens ein weiterer wasserlöslicher Feststoff in dem Behälter gehalten wird, wobei der wenigstens eine weitere wasserlösliche Feststoff ein Molekulargewicht oberhalb der Molekulargewichtsausschlußgrenze der Membran hat.

4. Verfahren nach Anspruch 1, 2 oder 3, dadurch gekennzeichnet, daß wenigstens einer der wasserlöslichen Feststoffe ein Zucker, ein wasserlösliches Glukosepolymer, ein wasserlösliches Polysaccharid, Reispulver, ein Elektrolytsalz oder eine Aminosäure ist.

5. Verfahren nach Anspruch 4, dadurch gekennzeichnet, daß wenigstens einer der wasserlöslichen Feststoffe ein Glukose-, Dextrose- Frucht- oder Saccharose-Zucker ist.

6. Verfahren nach einem der vorhergehenden Ansprüche, dadurch gekennzeichnet, daß die Membran mit fließendem Wasser in Berührung gebracht wird.

7. Verfahren nach Anspruch 6, dadurch gekennzeichnet, daß die Membran mit fließendem Wasser ausreichend lang in Berührung gebracht wird, daß im wesentlichen alle wasserlöslichen Feststoffe, die ein Molekulargewicht unterhalb der Mulekulargewichtssausschlußgrenze der Membran haben, aus dem Behälter herausdiffundieren.

8. Verfahren nach Anspruch 6, dadurch gekennzeichnet, daß alle wasserlöslichen Feststoffe ein Molekulargewicht unterhalb der Molekulargewichtsausschlußgrenze der Membran haben und daß die Membran mit fließendem Wasser ausreichend lang in Berührung gebracht wird, daß im wesentlichen alle wasserlöslichen Feststoffe aus dem Behälter herausdiffundieren, um Trinkwasser zu erzeugen, daß im wesentlichen frei von wasserlöslichen Feststoffen ist.

9. Verfahren nach einem der Ansprüche 1 bis 5, dadurch gekennzeichnet, daß die Membran mit einem vorbestimmten Volumen ruhigen oder umgerührten Wasser in Berührung gebracht wird.

10. Verfahren nach Anspruch 9, dadurch gekennzeichnet, daß die Membran mit Wasser ausreichend lang in Berührung gebracht wird, daß wenigstens einer der wasserlöslichen Feststoffe, der ein Molekulargewicht unterhalb der Molekulargewichtssausschlußgrenze hat, aus dem Behälter herausdiffundiert, bis die Feststoffkonzehtration innerhalb des Behälters im Gleichgewicht mit der Konzentration außerhalb des Behälters ist.

11. Verfahren nach einem der Ansprüche 1 bis 5, 9 oder 10, dadurch gekennzeichnet, daß die wasserlöslichen Feststoffe ein Gemisch bilden, das zur Erzeugung eines Ansatzes für eine orale Wiederhydrierungstherapie geeignet ist, und die Membran mit dem Wasser eine vorbestimmte Zeitdauer in Berührung gebracht wird, um den Ansatz für die orale Wiederhydrierungstherapie innerhalb des Behälter zu bilden.

12. Verfahren nach einem der Ansprüche 1 bis 5, 9 oder 10, dadurch gekennzeichnet, daß es eine Arzneimittellösung für die Injektion erzeugt, die vorzugsweise frei von Endotoxinen und Pyrogenen ist.

13. Verfahren nach einem der Ansprüche 1 bis 5, 9 oder 10, dadurch gekennzeichnet, daß es zur Wiederhydrierung von Trockenblutprodukten oder zur Erzeugung einer Blut- oder Plasmaersatzlösung für intravenöse Infusion, die vorzugsweise frei von Endotoxinen und Pyrogenen ist, verwendet wird.

14. Verfahren nach einem der Ansprüche 1 bis 5, 9 oder 10, dadurch gekennzeichnet, daß das Verfahren zur Erzeugung einer Nährmittelsuspension verwendet wird, wobei im Behälter eine getrocknete Nährmittelsubstanz, Trockenmilch oder teilentwässerte Lebensmittelsubstanz, die wenigstens einen wasserlöslichen Feststoff enthält, mit der Membran in Berührung gebracht wird.

15. Verfahren zur Wiederhydrierung getrockneter Blutprodukte oder zum Erzeugen einer Blut- oder Plasmaersatzlösung, die im wesentlichen frei von Mikroorganismen ist, für intravenöse Infusion, umfassend die Schritte:
Halten der Feststoffe aus Blutprodukten oder Blut- oder Plasmaersatz oder eine konzentrierte Lösung davon in einem Behälter, der mit einer selektiv permeablen Membran versehen ist, die mit dem Feststoff oder der Lösung in Berührung ist, wobei die Feststoffe oder deren konzentrierte Lösung wenigstens einen wasserlöslichen Feststoff enthalten, der ein Molekulargewicht hat, das unterhalb der Molekulargewichtsausschlußgrenze der Membran liegt; und
Inberührungbringen der entgegengesetzten Seite der Membran mit Wasser über eine vorbestimmte minimale Zeitdauer, um es dem Wasser zu ermöglichen, durch Osmose in den Behälter einzudringen, jedoch den Durchtritt von Mikroorganismen und vorzugsweise Endotoxinen und Pyrogenen aus dem Wasser in den Behälter zu unterbinden.

16. Verfahren zum Herstellen einer von Mikroorganismen im wesentlichen freien Arzneimittellösung für Injektionszwecke, umfassend die Schritte:
Halten der Arzneimittelfeststoffe oder einer konzentrierten Lösung davon in einem Behälter, der mit einer selektiv permeablen Membran versehen ist, die mit dem Feststoff oder der Lösung in Berührung ist, wobei die Feststoffe oder deren konzentrierte Lösung wenigstens einen wasserlöslichen Feststoff enthalten, der ein Molekulargewicht aufweist, das unterhalb der Molekulargewichtsausschlußgrenze der Membran liegt; und
Inberührungbringen der entgegengesetzten Seite der Membran mit Wasser über eine vorbestimmte minimale Zeitdauer, um es dem Wasser zu ermöglichen, durch Osmose in den Behälter einzudringen, jedoch den Durchgang von Mikroorganismen und vorzugsweise Endotoxinen und Pyrogenen aus dem Wasser in den Behälter zu verhindern.

17. Verfahren zum Erzeugen von Wasser für medizinische Zwecke, insbesondere zum Aufbereiten und Verabreichen von Medikamenten durch Injektion oder intravenöse Infusion, enthaltend die Schritte:
Halten wenigstens eines wasserlöslichen Feststoffs oder einer konzentrierten Lösung davon in einem Behälter, der mit einer selektiv permeablen Membran versehen ist, die mit dem Feststoff oder der Lösung in Berührung ist, wobei der wenigstens eine Feststoff oder die konzentrierte Lösung davon wenigstens einen wasserlöslichen Feststoff enthält, die ein Molekulargewicht unterhalb der Molekulargewichtsausschlußgrenze der Membran hat; und
Inberührungbringen der entgegengesetzten Seite der Membran mit Wasser über eine vorbestimmte minimale Zeitdauer, um es dem Wasser zu ermöglichen, durch Osmose in den Behälter einzudringen, jedoch den Durchgang von Mikroorganismen und vorzugsweise Endotoxinen und Pyrogenen aus dem mit der Membran in Berührung gebrachten Wasser in den Behälter zu verhindern.

18. Verfahren zum Erzeugen einer Lösung für orale Wiederhydrierungstherapie aus wasserlöslichen Feststoffen, die im wesentlichen frei von Mikroorganismen ist, enthaltend die Schritte:
Halten der Feststoffe oder einer konzentrierten Lösung davon in einem Behälter, der mit einer selektiv permeabien Membran versehen ist, die mit den Feststoffen oder ihrer konzentrierten Lösung in Berührung ist, und Inberührungbringen der entgegengesetzten Seite der Membran mit Wasser über eine vorbestimmte minimale Zeitdauer, um es dem Wasser zu ermöglichen, durch Osmose in den Behälter einzudringen, jedoch den Durchgang von Mikroorganismen aus dem Wasser in den Behälter zu verhindern, dadurch gekennzeichnet, daß
die wasserlöslichen Feststoffe dazu eingerichtet sind, eine orale Wiederhydrierungstherapielösung zu erzeugen;
wenigstens einer der wasserlöslichen Feststoffe aus Elektrolytsalzen ausgewählt ist, die ein Molekulargewicht unterhalb der Molekulargewichtsausschlußgrenze der Membran aufweisen;
eine vorbestimmte Menge der wasserlöslichen Feststoffe in den Behälter eingebracht wird; und
die Membran mit einem vorbestimmten Volumen ruhigen oder umgerührten Wassers in Berührung gebracht wird.

19. Verfahren nach Anspruch 18, dadurch gekennzeichnet, daß ein weiterer der wasserlöslichen Feststoffe ein Zucker, einwasserlösliches Glukosepolymer, ein wasserlösliches Polysaccharid oder Reispulver ist.

20. Verfahren nach einem der vorhergehenden Ansprüche, dadurch gekennzeichnet, daß die Membran aus Zellulose, Zellophan, Benzoylzellulose, Viskosezellulose oder Kollagen besteht.

21. Behälter zur Verwendung bei der Herstellung von Lösungen nach dem Verfahren von Anspruch 1, mit einem ersten Abteil (6) mit einer selektiv permeablen Membran, das den Feststoff (8) oder eine konzentrierte Lösung davon enthält, dadurch gekennzeichnet, daß
der Behälter ein zweites Abteil (5) enthält,
daß die ersten und zweiten Abteile durch ein Bauelement (7) voneinander getrennt sind, daß die selektiv permeable Membran aufweist, und
daß der wenigstens eine wasserlösliche Feststoff ein Molekulargewicht unterhalb der Molekulargewichtsausschlußgrenze der Membran hat.

22. Behälter nach Anspruch 21, dadurch gekennzeichnet, daß die Membran aus Zellulose, Zellophan, Benzoylzellulose, Viskosezellulose oder Kollagen besteht.

23. Behälter zur Verwendung bei der Erzeugung einer oralen Wiederhydrierungstherapielösung durch das Verfahren nach Anspruch 18, enthaltend:
ein erstes Abteil (6) mit einer selektiv permeablen Membran, das die vorbestimmte Menge der wasserlöslichen Feststoffe (8) oder eine konzentrierte Lösung davon aufnimmt, dadurch gekennzeichnet, daß der Behälter enthält:
ein zweites Abteil (5) zur Aufnahme einer vorbestimmten Menge Wassers,
eine Einrichtung (3) zum Einleiten des Wassers in das zweite Abteil,
ein Bauelement (7), das die selektiv permeable Membran aufweist enthält, die die ersten und zweiten Abteile voneinander trennt, und
eine Einrichtung (4) zum Abziehen der Lösung aus dem ersten Abteil, und
daß der wenigstens eine wasserlösliche Feststoff ein Molekulargewicht unterhalb der Molekulargewichtsausschlußgrenze der Membran hat.

24. Behälter nach Anspruch 21, 22 oder 23, dadurch gekennzeichnet, daß das erste Abteil mehrere sprengbare Kammern (19) aufweist, die jeweils eine vorbestimmte Menge der wasserlöslichen Feststoffe oder eine konzentrierte Lösung davon enthalten, wobei der Behälter wiederverwendbar ist, nachdem eine erste der Kammern gesprengt worden ist, um seinen Inhalt in das erste Abteil zu bringen, Wasser in das erste Abteil zur Bildung einer Lösung eingedrungen ist und daraus entleert worden ist.

25. Behälter zur Erzeugung von nicht-toxischem Trinkwasser, das im wesentlichen frei von Mikroorganismen ist, durch das Verfahren nach Anspruch 6, wobei der Behälter eine selektiv permeable Membran aufweist und wenigstens einen mit der Membran in Berührung befindlichen wasserlöslichen Feststoff oder eine konzentrierte Lösung davon enthält, dadurch gekennzeichnet, daß jeder oder alle der Feststoffe ein Molekulargewicht unterhalb der Molekulargewichtsausschlußgrenze der Membran aufweist bzw. aufweisen.

26. Behälter nach einem der Ansprüche 21 bis 25, dadurch gekennzeichnet, daß das erste Abteil oder das zweite Abteil Einrichtungen zum Anzeigen aufweisen, wann die Lösung ihren beabsichtigten Ansatz erreicht hat.

27. Behälter zum Erzeugen einer Nährmittelsuspension, die im wesentlichen frei von Mikroorganismen ist, durch das Verfahren nach Anspruch 14, dadurch gekennzeichnet, daß sich in dem Behälter eine getrocknete Nährmittelsubstanz, Milchpartikel oder teilentwässerte Lebensmittelsubstanz befindet, die wenigstens einen wasserlöslichen Feststoff enthalten und mit der Membran in Berührung sind, wobei der oder die wasserlöslichen Feststoff(e) ein Molekulargewicht unterhalb der Molekulargewichtsausschlußgrenze der Membran hat bzw. haben.

28. Behälter zum Wiederhydrieren getrockneter Blutprodukte oder zum Erzeugen einer Blut- oder Plasmaersatzlösung, die im wesentlichen frei von Mikroorganismen und vorzugsweise Endotoxinen und Pyrogenen ist, für die intravenöse Infusion, durch das Verfahren nach Anspruch 15, wobei der Behälter eine selektiv permeable Membran aufweist und die Feststoffe der Blutprodukte oder des Blut- oder Plasmaersatzes oder eine konzentrierte Lösung davon und wenigstens einen wasserlöslichen Feststoff enthält, der ein Molekulargewicht unterhalb der Molekulargewichtsausschlußgrenze der Membran hat und mit der Membran in Berührung ist.

29. Behälter zum Erzeugen einer von Mikroorganismen und vorzugsweise Endotoxinen und Pyrogenen freien Arzneimittellösung für die Injektion durch das Verfahren nach Anspruch 16, wobei der Behälter eine selektiv permeable Membran hat und lösbare Feststoff(e) der Arznei oder eine konzentrierte Lösung davon in Berührung mit der Membran enthält, wobei wenigstens einer der löslichen Feststoff ein Molekulargewicht unterhalb der Molekulargewichtsausschlußgrenze der Membran hat.

30. Behälter zum Erzeugen eines Wassers für medizinische Zwecke, insbesondere zum Aufbereiten und Verabreichen von Arzneimitteln durch Injektion oder intravenöse Infusion, die im wesentlichen frei von Mikroorganismen und vorzugsweise Endotoxinen und Pyrogenen ist, durch das Verfahren nach Anspruch 17, wobei der Behälter eine selektiv permeable Membran aufweist und den wenigstens einen wasserlöslichen Feststoff oder eine konzentrierte Lösung davon in Berührung mit der Membran enthält, und der wenigstens eine wasserlösliche Feststoff ein Molekulargewicht unterhalb der Molekulargewichtsausschlußgrenze der Membran hat.

31. Verwendung eines Behälters nach einem der Ansprüche 1 bis 26, 29 oder 30 für die Zubereitung eines Trinkwassers oder einer trinkbaren Lösung oder eines Medikaments zur Verwendung bei der Behandlung des menschlichen oder tierischen Körpers oder von Wasser zur Aufbereitung und Verabreichung eines Medikaments durch Injektion oder Infusion, wobei die Zubereitung nach dem Verfahren 1, 16 oder 17 erfolgt.

## Revendications

1. Procédé pour la production d'une solution non-toxique d'au moins un solide soluble dans l'eau substantiellement dépourvue de microorganismes, ledit procédé comprenant la rétention du solide ou d'une solution concentrée de celui-ci dans un récipient muni d'une membrane sélectivement perméable en contact avec le solide ou la solution et la mise en contact de la face opposée de la membrane avec de l'eau pendant une période de temps minimum prédéterminée de manière à permettre à l'eau de pénétrer dans le récipient par osmose, mais à empêcher le passage de microorganismes de l'eau dans le récipient, caractérisé en ce que ledit au moins un solide soluble dans l'eau a un poids moléculaire inférieur au poids moléculaire limite de la membrane.

2. Procédé selon la revendication 1, caractérisé en ce que la membrane a un poids moléculaire limite tel que les microorganismes et les entérotoxines des espèces de V.Cholerae et Shigella sont empêchés de pénétrer dans le récipient.

3. Procédé selon la revendication 1 ou la revendication 2, caractérisé en ce qu'au moins un autre solide soluble dans l'eau est retenu dans le récipient, ledit au moins un autre solide soluble dans l'eau ayant un poids moléculaire supérieur au poids moléculaire limite de la membrane.

4. Procédé selon la revendication 1, la revendication 2 ou la revendication 3, caractérisé en ce qu'au moins un des solides solubles dans l'eau est choisi parmi les sucres, les polymères de glucose solubles dans l'eau, les polysaccharides solubles dans l'eau, la poudre de riz, les sels électrolytes et les acides aminés.

5. Procédé selon la revendication 4, caractérisé en ce qu'au moins un des solides solubles dans l'eau est un sucre choisi parmi le glucose, le dextrose, le fructose et le sucrose.

6. Procédé selon l'une quelconque des revendications précédentes, caractérisé en ce que la membrane est mise en contact avec de l'eau en circulation.

7. Procédé selon la revendication 6, caractérisé en ce que la membrane est mise en contact avec de l'eau courante pendant un temps suffisant pour que substantiellement tous les solides solubles dans l'eau ayant un poids moléculaire inférieur au poids moléculaire limite de la membrane diffusent en dehors du récipient.

8. Procédé selon la revendication 6, caractérisé en ce que tous les solides solubles dans l'eau ont un poids moléculaire inférieur au poids moléculaire limite de la membrane et en ce que la membrane est mise en contact avec de l'eau en circulation pendant un temps suffisant pour que substantiellement tous les solides solubles dans l'eau diffusent en dehors du récipient pour la production d'eau potable substantiellement dépourvue de solides solubles dans l'eau.

9. Procédé selon l'une quelconque des revendications 1 à 5 , caractérisé en ce que la membrane est mise en contact avec un volume prédéterminé d'eau statique ou agitée.

10. Procédé selon la revendication 9, caractérisé en ce que la membrane est mise en contact avec de l'eau pendant un temps suffisant pour qu'au moins un des solides solubles dans l'eau ayant un poids moléculaire inférieur au poids moléculaire limite diffuse en dehors du récipient jusqu'à ce que la concentration du solide à l'intérieur du récipient soit en équilibre avec la concentration à l'extérieur du récipient.

11. Procédé selon l'une quelconque des revendications 1 à 5, ou la revendication 9 ou la revendication 10, caractérisé en ce que les solides solubles dans l'eau constituent un mélange convenant pour la production d'une formulation pour un traitement oral de réhydratation et en ce que la membrane est mise en contact avec l'eau pendant un temps prédéterminé de manière à former dans le récipient une formulation pour un traitement oral de réhydratation.

12. Procédé selon l'une quelconque des revendications 1 à 5, ou la revendication 9 ou la revendication 10, caractérisé en ce que le procédé fournit une solution pour injection d'un médicament, de préférence dépourvue d'endotoxines et de substances pyrogènes.

13. Procédé selon l'une quelconque des revendications 1 à 5, ou la revendication 9 ou la revendication 10, caractérisé en ce que le procédé est utilisé pour la réhydratation de produits sanguins séchés ou la production d'une solution de substitut de sang ou de plasma pour perfusion intraveineuse, de préférence dépourvus d'endotoxines et de substances pyrogènes.

14. Procédé selon l'une quelconque des revendications 1 à 5, ou la revendication 9 ou la revendication 10, pour la production d'une suspension nutritionnelle, le procédé comprenant la rétention dans le récipient d'une substance nutritionnelle sèche, de solides de lait ou de substances nutritives partiellement déshydratées, contenant au moins un solide soluble dans l'eau en contact avec la membrane.

15. Procédé pour la réhydratation de produits sanguins ou pour la production de solution de substitut de sang ou de plasma substantiellement dépourvus de microorganismes pour perfusion intraveineuse, le procédé comprenant:
la rétention des solides des produits sanguins ou du substitut de sang ou de plasma ou d'une solution concentrée de ceux-ci dans un récipient muni d'une membrane sélectivement perméable en contact avec le solide ou la solution, les solides ou la solution concentrée de ceux-ci comprenant au moins un solide soluble dans l'eau qui a un poids moléculaire inférieur au poids moléculaire limite de la membrane; et
la mise en contact de la face opposée de la membrane avec de l'eau pour une période de temps minimum prédéterminée de manière à permettre à l'eau de pénétrer dans le récipient par osmose, mais à empêcher le passage dans le récipient des microorganismes, et de préférence des endotoxines et des substances pyrogènes, de l'eau.

16. Procédé pour la production d'une solution pour injection d'un médicament substantiellement dépourvue de microorganismes, le procédé comprenant:
la rétention des solides du médicament ou d'une solution concentrée de ceux-ci dans un récipient muni d'une membrane sélectivement perméable en contact avec le solide ou la solution, les solides ou la solution concentrée de ceux-ci comprenant au moins un solide soluble dans l'eau qui a un poids moléculaire inférieur au poids moléculaire limite de la membrane; et
la mise en contact de la face opposée de la membrane avec de l'eau pendant une période de temps minimum prédéterminée de manière à permettre à l'eau de pénétrer dans le récipient par osmose, mais à empêcher le passage dans le récipient de microorganismes, et de préférence des endotoxines et des substances pyrogènes, de l'eau.

17. Procédé pour la production d'eau à usage médical, en particulier pour la fabrication et l'administration de médicaments par injection ou perfusion intraveineuse, le procédé comprenant:
la rétention d'au moins un solide soluble dans l'eau ou d'une solution concentrée de celui-ci dans un récipient muni d'une membrane sélectivement perméable en contact avec le solide ou la solution, le(s) solide(s) ou la solution concentrée de celui-ci (ceux-ci) comprenant au moins un solide soluble dans l'eau qui a un poids moléculaire inférieur au poids moléculaire limite de la membrane; et
la mise en contact de la face opposée de la membrane avec de l'eau pendant une période de temps minimum prédéterminée de manière à permettre à l'eau de pénétrer dans le récipient par osmose, mais à empêcher le passage dans le récipient de microorganismes, et de préférence des endotoxines et des substances pyrogènes, de l'eau avec laquelle la membrane est mise en contact.

18. Procédé pour la production d'une solution pour traitement oral de réhydratation substantiellement dépourvue de microorganismes, ledit procédé comprenant la rétention des solides ou d'une solution concentrée de ceux-ci dans un récipient muni d'une membrane sélectivement perméable en contact avec les solides ou leur solution concentrée et la mise en contact de la face opposée de la membrane avec de l'eau pendant une période de temps minimum prédéterminée de manière à permettre à l'eau de pénétrer dans le récipient par osmose, mais à empêcher le passage dans le récipient des microorganismes de l'eau, caractérisé en ce que:
les solides solubles dans l'eau sont adaptés à la production d'une solution pour traitement oral de réhydratation;
au moins un des solides solubles dans l'eau est sélectionné parmi les sels électrolytes ayant un poids moléculaire inférieur au poids moléculaire limite de la membrane;
une quantité prédéterminée des solides solubles dans l'eau est incluse dans le récipient; et
la membrane est mise en contact avec un volume prédéterminé d'eau statique ou agitée.

19. Procédé selon la revendication 18, caractérisé en ce qu'un autre des solides solubles dans l'eau est choisi parmi les sucres, les polymères de glucose solubles dans l'eau, les polysaccharides solubles dans l'eau et la poudre de riz.

20. Procédé selon l'une quelconque des revendications précédentes caractérisé en ce que la membrane est choisie parmi la cellulose, la cellophane, la cellulose benzoylée, la cellulose viscose et le collagène.

21. Récipient pour utilisation dans la production de solutions selon le procédé de la revendication 1, ledit récipient ayant un premier compartiment (6) avec une membrane sélectivement perméable et contenant le solide (8) ou une solution concentrée de celui-ci caractérisé en ce que le récipient comprend un deuxième compartiment (5), en ce que le premier et le deuxième compartiment sont séparés par un membre (7) contenant la membrane sélectivement perméable et en ce que ledit au moins un solide soluble dans l'eau a un poids moléculaire inférieur au poids moléculaire limite de la membrane.

22. Récipient selon la revendication 21, caractérisé en ce que la membrane est choisie parmi la cellulose, la Cellophane, la cellulose benzoylée, la cellulose viscose et le collagène.

23. Récipient pour utilisation dans la production d'une solution de traitement oral de réhydratation selon le procédé de la revendication 18, le récipient ayant un premier compartiment (6) avec une membrane sélectivement perméable et contenant la quantité prédéterminée des solides (8) solubles dans l'eau ou une solution concentrée de ceux-ci, caractérisé en ce que en ce que le récipient comprend: un deuxième compartiment (5) pour contenir une quantité prédéterminée d'eau, des moyen (3) pour l'introduction de l'eau dans le deuxième compartiment, un membre (7) contenant la membrane sélectivement perméable séparant le premier et le deuxième compartiment et des moyens (4) pour le retrait de ladite solution du premier compartiment et en ce que ledit au moins un solide soluble dans l'eau a un poids moléculaire inférieur au poids moléculaire limite de la membrane.

24. Récipient selon la revendication 21 , la revendication 22 ou la revendication 23, caractérisé en ce que le premier compartiment comprend une pluralité de compartiments crevables (10), chacun contenant une quantité prédéterminée de solides solubles dans l'eau ou une solution concentrée de ceux-ci, le récipient étant réutilisable après qu'un premier des compartiments ait été crevé pour introduire son contenu dans le premier compartiment, que l'eau ait filtré dans le premier compartiment pour former une solution et qu'elle en ait été retirée.

25. Récipient pour la production d'eau potable non toxique substantiellement dépourvue de microorganismes selon le procédé de la revendication 6, ledit récipient ayant une membrane sélectivement perméable et contenant, au contact de la membrane, le solide ou les solides solubles dans l'eau ou une solution concentrée de ceux-ci, caractérisé en ce que ledit au moins un ou tous les solides ont un poids moléculaire inférieur au poids moléculaire limite de la membrane.

26. Récipient selon l'une quelconque des revendications 21 à 25 caractérisé en ce que le premier compartiment ou le second compartiment comprend des moyens pour indiquer quand la solution a atteint la formulation prévue.

27. Récipient pour la production d'une suspension nutritionnelle substantiellement dépourvue de microorganismes par le procédé de la revendication 14 caractérisé en ce que le récipient contient une substance nutritionnelle séchée, des solides de lait ou des substances nutritives partiellement déshydratées contenant au moins un solide soluble dans l'eau, en contact avec la membrane, le ou un des solide(s) soluble(s) dans l'eau ayant un poids moléculaire inférieur au poids moléculaire limite de la membrane.

28. Récipient pour la réhydratation de produits sanguins séchés ou pour la production d'une solution de substitut de sang ou de plasma substantiellement dépourvus de microorganismes, et de préférence d'endotoxines et de substances pyrogènes, pour perfusion intraveineuse selon le procédé de la revendication 15, le récipient ayant une membrane sélectivement perméable et contenant en contact avec la membrane les solides des produits sanguins ou du substitut de sang ou de plasma ou une solution concentrée de ceux-ci et au moins un solide soluble dans l'eau ayant un poids moléculaire inférieur au poids moléculaire limite de la membrane.

29. Récipient pour la production d'une solution pour injection de médicaments substantiellement dépourvue de microorganismes et de préférence d'endotoxines et de substances pyrogènes selon le procédé de la revendication 16, le récipient ayant une membrane sélectivement perméable et contenant le(s) solide(s) soluble(s) du médicament ou une solution concentrée de ceux-ci en contact avec la membrane, au moins un des solide(s) soluble(s) ayant un poids moléculaire inférieur au poids moléculaire limite de la membrane.

30. Récipient pour la production d'eau à usage médical, en particulier pour la fabrication et l'administration de médicaments par injection ou perfusion intraveineuse substantiellement dépourvue de microorganismes et de préférence d'endotoxines et de substances pyrogènes selon le procédé de la revendication 17, le récipient ayant une membrane sélectivement perméable et contenant ledit au moins un solide soluble dans l'eau ou une solution concentrée de celui-ci en contact avec la membrane, ledit au moins un solide soluble dans l'eau ayant un poids moléculaire inférieur au poids moléculaire limite de la membrane.

31. Utilisation d'un récipient selon l'une quelconque des revendications 21 à 26 ou la revendication 29 ou la revendication 30 pour la préparation d'eau potable ou d'une solution potable ou d'un médicament pour utilisation dans le traitement du corps humain ou animal ou d'eau pour la fabrication et l'administration d'un médicament par injection ou perfusion, respectivement selon la méthode des revendications 1, 16 ou 17.
